# EUROPÄISCHE PATENTANMELDUNG

(11) **EP 3 858 224 A1**
(43) Veröffentlichungstag der Anmeldung: **04.08.2021**
(21) Anmeldenummer: 20154711.4
(22) Anmeldetag: 30.01.2020
(51) Int. Cl.: A61B 3/10, A61B 3/15

(54) **VERFAHREN UND VORRICHTUNG ZUM AUSRICHTEN EINES AUGES RELATIV ZU EINER GERÄTEACHSE**

(71) Anmelder: Visotec GmbH, 23552 Lübeck (DE)
(72) Erfinder: KOCH, Peter, 23558 Lübeck (DE); MÜNST, Michael, 23552 Lübeck (DE); SUDKAMP, Helge, 23552 Lübeck (DE); MOLTMANN, Moritz, 23552 Lübeck (DE); THEISSEN-KUNDE, Dirk, 23562 Lübeck (DE); HÜTTMANN, Gereon, 23564 Lübeck (DE)
(74) Vertreter: Glawe, Delfs, Moll

(57) **Zusammenfassung**

Verfahren zum Ausrichten eines Auges (14) relativ zu einer Geräteachse (17), bei dem ein Fixierlicht (25) erzeugt wird, so dass das Fixierlicht (25) sich koaxial zu der Geräteachse (17) ausbreitet. Es wird ein Peillicht (18) erzeugt, so dass das Peillicht (18) sich koaxial zu der Geräteachse (17) ausbreitet und dass das Peillicht (18) eine von der Retina des Auges (14) beabstandete Fokuslage hat. Das Auge (14) wird in radialer Richtung (12) relativ zu der Geräteachse (17) bewegt, so dass mit dem Auge (14) eine Umrandung (29) des Peillichts (18) und das Fixierlicht (25) konzentrisch wahrgenommen werden. Die Erfindung betrifft auch eine entsprechende Vorrichtung.

## Beschreibung

Die Erfindung betrifft ein Verfahren und eine Vorrichtung zum Ausrichten eines Auges relativ zu einer Geräteachse.

Es gibt verschiedene Anwendungen und Untersuchungsverfahren, bei denen ein Auge zu einer Geräteachse ausgerichtet wird. Dazu gehören Messungen mittels optischer Kohärenztomographie (OCT), mit denen der hintere Augenabschnitt eines Patientenauges untersucht wird. Bislang werden solche Untersuchungen üblicherweise unter der Aufsicht von medizinischem Fachpersonal durchgeführt. Das medizinische Fachpersonal ist geschult und kann sicherstellen, dass das Patientenauge während der Untersuchung richtig zu dem Gerät ausgerichtet ist.

Zukünftig wird es Anwendungsbereiche geben, bei denen der Patient OCT-Messungen am Auge eigenhändig vornimmt, DE 10 2015 113 465 A1, EP 3 517 021 A1. Die Messung wird dann durchgeführt, ohne dass eine weitere Person zur Verfügung steht, die auf die richtige Ausrichtung zwischen Patientenauge und Gerät achten kann.

Der Erfindung liegt die Aufgabe zugrunde, ein Verfahren und eine Vorrichtung vorzustellen, mit denen das Ausrichten eines Auges relativ zu einer Geräteachse erleichtert wird. Ausgehend vom genannten Stand der Technik wird die Aufgabe gelöst mit den Merkmalen der unabhängigen Ansprüche. Vorteilhafte Ausführungsformen sind in den Unteransprüchen angegeben.

Bei dem erfindungsgemäßen Verfahren wird ein Fixierlicht erzeugt, so dass das Fixierlicht sich koaxial zu der Geräteachse ausbreitet. Es wird ein Peillicht erzeugt, so dass das Peillicht sich koaxial zu der Geräteachse ausbreitet und dass das Peillicht eine von der Retina des Auges beabstandete Fokuslage hat. Das Auge wird in radialer Richtung relativ zu der Geräteachse bewegt, so dass mit dem Auge eine Umrandung des Peillichts und das Fixierlicht konzentrisch wahrgenommen werden.

Ein Auge kann im Allgemeinen zu einer Achse ausgerichtet werden, indem zwei Objekte in einem axialen Abstand auf der Achse angeordnet werden und das Auge in radialer Richtung bewegt wird, bis die beiden Objekte zueinander ausgerichtet sind. Dieses Prinzip ist beispielsweise aus der Schifffahrt bekannt. Auf das Ausrichten eines Auges relativ zu einer Geräteachse lässt sich dieses Prinzip nicht ohne weiteres übertragen, weil die Ausdehnung des Geräts in axialer Richtung in aller Regel nicht groß genug ist, um die Objekte mit einem so großen axialen Abstand anordnen zu können, dass eine Ausrichtung mit hinreichender Genauigkeit möglich wird.

Die Erfindung basiert auf dem Gedanken, dem Auge zwei Orientierungsmarken anzubieten, so dass für das Auge ein großer axialer Abstand zwischen den Orientierungsmarken zu bestehen scheint. Die Orientierungsmarken werden gebildet einerseits durch das Fixierlicht, das für das Auge in weiter Ferne zu sein scheint, und andererseits durch das Peillicht, das für das Auge einen geringeren Abstand zu haben scheint. Ein Strahlenbündel, dessen Fokuslage von der Retina des Auges beabstandet ist, wird vom Auge nicht scharf wahrgenommen. Mit der Erfindung wird vorgeschlagen, eine Umrandung des Peillichts als Marke für die Ausrichtung zu verwenden. Eine solche Umrandung kann das Auge auch dann scharf wahrnehmen, wenn das Peillicht einen mit im Wesentlichen gleichmäßiger Helligkeit ausgeleuchteten Fleck auf der Retina bildet. Die Umrandung des Peillichts und das Fixierlicht scheinen für das Auge einen großen axialen Abstand zueinander zu haben, so dass eine präzise Ausrichtung des Auges relativ zu der Geräteachse ermöglicht wird.

Das Fixierlicht kann ein kollimierter Lichtstrahl sein, der auf das Auge gerichtet wird und den der Benutzer als punktförmiges Licht wahrnimmt. In einer Ausführungsform ist das Fixierlicht so gestaltet, dass es ins Unendliche abgebildet und dann auf das Auge gerichtet wird. So wird das Objekt als in weiter Entfernung liegend wahrgenommen wird, wenn es durch die optischen Elemente im vorderen Augenabschnitt auf die Retina abgebildet wird. Die Strahlenbündel des Fixierlichts treffen dann im Wesentlichen kollimiert auf das Auge.

Das Objekt des Fixierlichts kann eine Punktquelle oder ein auf einem Display oder Dia dargestelltes Objekt sein. Zwischen dem Objekt des Fixierlichts und dem Auge wird in der Regel eine Abbildungsoptik angeordnet sein, so dass ausgehend von allen Objektpunkten ein im Wesentlichen kollimiertes Strahlenbündel auf das Auge trifft. Bei der Abbildungsoptik kann es sich um eine einzelne Linse handeln. Das Objekt kann eine für eine Orientierungsmarke geeignete Form haben und beispielsweise ein Kreuz, ein Kreis oder gegebenenfalls auch nur ein einzelner Punkt sein. Ein Mittelpunkt des Objekts kann mit der Geräteachse zusammenfallen.

Ein ins Unendliche abgebildetes Objekt wird von einem auf unendlich weit entfernte Objekte adaptierten nicht-fehlsichtigen Auge scharf wahrgenommen. Wünschenswert ist, dass das Fixierlicht auch für fehlsichtige Patienten gut sichtbar ist. Dafür ist es von Vorteil, wenn die numerische Apertur, mit der das Objekt nach unendlich abgebildet wird, klein ist. Insbesondere kann die numerische Apertur kleiner sein als 0,03, vorzugsweise kleiner sein als 0,02, weiter vorzugsweise kleiner sein als 0,015. Wird beispielsweise die numerische Apertur durch eine Aperturblende begrenzt, die zwischen dem Auge und der Abbildungsoptik angeordnet ist, mit der das Objekt nach unendlich abgebildet wird, so kann die Austrittspupille einen Durchmesser von weniger als 2 mm, vorzugsweise von weniger als 1 mm, weiter vorzugsweise von weniger als 0,5 mm haben. Auch bei Fehlsichtigkeit zwischen beispielsweise +3 dpt. und -3 dpt. wird auf diese Weise das Objekt scharf wahrgenommen.

Wenn das Licht eine von der Retina des Auges beabstandete Fokuslage hat, ist das Peillicht nicht auf die Retina fokussiert. Das bei Licht erzeugt also keine scharfe Abbildung auf der Retina. Die Begriffe Fokuslage und Fokuspunkt im Sinne der Erfindung bezieht sich auf den der Retina nächstgelegenen Fokuspunkt des Peillichts. Das Peillicht kann weitere vor oder hinter der Retina angeordnete Fokussierungen haben, die einen größeren Abstand zu der Retina haben als der nächstgelegene Fokuspunkt. Der Abstand zwischen der Retina und dem Fokuspunkt ist vorzugweise größer als 10 mm, weiter vorzugsweise größer als 20 mm, weiter vorzugsweise größer als 30 mm.

In einer Ausführungsform ist der Fokuspunkt des Peillichts vor der Cornea des Auges angeordnet, sodass das Peillicht in divergentem Zustand auf das Auge auftrifft. Der Fokuspunkt kann auf der Geräteachse liegen. Der Fokuspunkt kann zwischen der Cornea des Auges und der Abbildungsoptik des Fixierlichts angeordnet sein.

Der Fokuspunkt kann einen Abstand zwischen 10 mm und 25 mm, vorzugsweise zwischen 14 mm und 18 mm zu der Vorderseite des Auges haben. Dies entspricht der Brennweite der optischen Elemente des Auges, so dass das Peillicht als im Wesentlichen kollimiertes Strahlenbündel auf die Retina trifft. Das Peillicht bildet dann auf der Retina einen Fleck von im Wesentlichen gleichmäßiger Helligkeit. Möglich ist auch, dass das Peillicht auf der Retina eine strukturierte Verteilung hat, beispielsweise in Form von konzentrischen Kreisen. Eine gleichmäßige Helligkeit ist von Vorteil, wenn die Umrandung des Flecks als Orientierungsmarke dienen soll. Der Fleck auf der Retina hat vorzugsweise einen Durchmesser von wenigstens 1 mm, vorzugsweise von wenigstens 2 mm, weiter vorzugsweise von wenigstens 3 mm. Andererseits ist der Fleck auf der Retina vorzugsweise nicht größer als 5 mm, weiter vorzugsweise nicht größer als 4 mm. In einer Ausführungsform ist der divergente Strahlengang des Peillichts so stark aufgeweitet, dass die Pupille vollständig überdeckt wird und auch ein Teil der Iris mit dem Peillicht beleuchtet wird. In diesem Fall kann der Rand der Iris die mit dem Auge wahrgenommene Umrandung des Peillichts bilden.

Möglich ist auch, den Strahlengang des Peillichts so zu begrenzen, dass er nur einen Teil der Pupille ausfüllt. Zu diesem Zweck kann eine den Strahlengang des Peillichts begrenzende Blende vorgesehen sein. Die Blende kann insbesondere in einem Abschnitt des Strahlengangs angeordnet sein, in dem das Peillicht als kollimiertes Strahlenbündel vorliegt.

Möglich ist auch, dass der Fokuspunkt zwischen der Retina und der Vorderseite des Auges angeordnet ist. Dabei kann der Abstand zwischen der Retina und dem Fokuspunkt größer sein, vorzugsweise um wenigstens den Faktor zwei größer sein als der Abstand zwischen dem Fokuspunkt und der Vorderseite des Auges. Das Peillicht kann mit einer Lichtquelle erzeugt werden. Bei der Lichtquelle kann es sich beispielsweise um ein Austrittsende eines Lichtleiters handeln. Zwischen der Lichtquelle und dem Fokuspunkt des Peillichts kann ein wenigstens eine Linse umfassendes Linsensystem angeordnet sein. Das Linsensystem kann so gestaltet sein, dass das Peillicht in einem zwischen der Lichtquelle und dem Fokuspunkt angeordneten Abschnitt des Strahlengangs zu einem kollimierten Strahlenbündel geformt wird.

Die Umrandung des Peillichts kann erzeugt werden, indem im Strahlengang des Peillichts eine Blende angeordnet ist, sodass die Blende einen das Peillicht umgebenden Schatten auf die Retina wirft.

Die Umrandung des Peillichts kann beispielsweise die Form eines Kreises oder eines Polygons haben. Um das Auge zu der Geräteachse auszurichten, wird der Mittelpunkt der Umrandung mit dem Mittelpunkt des Fixierlichts zur Übereinstimmung gebracht. Hat das Fixierlicht die Form eines Punkts, so muss der Benutzer versuchen, den Punkt in der Mitte der Umrandung zu positionieren. Wenn dies nicht gelingt, wird die Ausrichtung ungenau.

Die Genauigkeit und Anwendungsfreundlichkeit können erhöht werden, indem das Fixierlicht von einem Mittelpunkt des dargestellten Objekts beabstandete Elemente umfasst, die in der Wahrnehmung des Benutzers nahe der Umrandung des Peillichts angeordnet sind, wenn das Auge richtig zur Geräteachse ausgerichtet ist. Dies ist beispielsweise der Fall, wenn das mit dem Fixierlicht dargestellte Objekt die Form eines Kreises oder eines Kreuzes hat, wo jeweils ein äußerer Rand ein peripheres Element bildet. Der Durchmesser der Umrandung und/oder der Durchmesser des mit dem Fixierlicht dargestellten Objekts können einstellbar sein. Insbesondere können diese so einstellbar sein, dass beide in der Wahrnehmung des Auges die gleiche Größe haben. Zwei gleich groß wahrgenommene Elemente können vom Benutzer leichter zur Überdeckung gebracht werden als zwei Elemente, die vom Benutzer verschiedenen groß wahrgenommen werden.

Zur leichten Unterscheidbarkeit kann die Wellenlänge des Fixierlichts von der Wellenlänge des Peillichts abweichen. Die Wellenlänge des Fixierlichts kann innerhalb des Wellenlängenbereichs des sichtbaren Lichts liegen. Als sichtbares Licht wird der Wellenlängenbereich zwischen 400 nm und 750 nm bezeichnet.

Die Wellenlänge des Peillichts kann innerhalb des Wellenlängenbereichs des sichtbaren Lichts liegen. Die Leistung des Peillichts wird dann so bemessen, dass das Peillicht für den Patienten gut wahrnehmbar ist, also beispielsweise im Bereich von einige µW liegt. Möglich ist auch, dass das Peillicht eine Wellenlänge hat, die außerhalb des sichtbaren Lichts liegt, wobei insbesondere die Wellenlänge des Peillichts größer sein kann als die Wellenlänge des Fixierlichts. Das Peillicht kann beispielsweise eine Wellenlänge zwischen 750 nm und 1100 nm, vorzugsweise zwischen 800 nm und 900 nm haben. Die Erfindung hat erkannt, dass das Peillicht, das auf der Retina einen im Wesentlichen mit gleichmäßige Helligkeit ausgeleuchteten Fleck bildet, vom Auge auch dann wahrgenommen wird, wenn die Wellenlänge des Peillichts außerhalb des Wellenlängenbereichs liegt, der gemeinhin als sichtbares Licht bezeichnet wird. Die scharfe Umrandung des hellen Flecks und das im sichtbaren Wellenlängenbereich liegende Fixierlicht können vom Benutzer gut wahrgenommen und zur Ausrichtung verwendet werden. Eine gute Wahrnehmbarkeit des Peillichts ergibt sich bei einer Wellenlänge zwischen 800 nm und 900 nm beispielsweise, wenn sich eine mittlere Lichtleistung zwischen 2 mW und 10 mW über einen Fleck mit 3 mm Durchmesser auf der Retina verteilt.

Das Gerät, zu dessen Geräteachse das Auge ausgerichtet wird, kann ein OCT-Gerät sein, das dazu ausgelegt ist, mittels optischer Kohärenztomografie Messwerte vom hinteren Augenabschnitt zu gewinnen. Insbesondere kann es sich um ein Full-Field-OCT-Gerät handeln, bei dem ein Bildsensor verwendet wird, der mit einer Aufnahme eine En-face-Bild des Augenhintergrunds aufnimmt. Das Verfahren kann so durchgeführt werden, dass das Auge nach dem Ausrichten relativ zu der Geräteachse für die Dauer einer OCT-Messung in Position gehalten wird.

Wenn das erfindungsgemäße Verfahren mit einem OCT-Gerät durchgeführt wird, kann zum Erzeugen des Peillichts dieselbe Lichtquelle verwendet werden, mit der auch das OCT-Licht erzeugt wird. Das OCT-Licht hat dann einen doppelten Nutzen.

Die Erfindung betrifft außerdem eine Vorrichtung zum Ausrichten eines Auges relativ zu einer Geräteachse. Die Vorrichtung umfasst eine erste Lichtquelle zum Erzeugen eines Peillichts, wobei der Strahlengang des Peillichts sich von der ersten Lichtquelle über einen Fokuspunkt zu dem Auge erstreckt, so dass das Peillicht eine von der Retina beabstandete Fokuslage hat. Die Vorrichtung umfasst eine zweite Lichtquelle zum Erzeugen eines Fixierlichts, so dass das Fixierlicht als kollimierter Strahlengang auf das Auge trifft. Die zweite Lichtquelle kann ein Display sein, auf dem ein Objekt dargestellt ist.

Die Vorrichtung kann mit weiteren Merkmalen fortgebildet werden, die im Zusammenhang des erfindungsgemäßen Verfahrens beschrieben sind. Das Verfahren kann mit weiteren Merkmalen fortgebildet werden, die im Zusammenhang erfindungsgemäßen Vorrichtung beschrieben sind.

Die Erfindung wird nachfolgend unter Bezugnahme auf die beigefügten Zeichnungen anhand vorteilhafter Ausführungsformen beispielhaft beschrieben. Es zeigen:
- Fig. 1:: eine schematische Darstellung einer erfindungsgemäßen Vorrichtung;
- Fig. 2:: einen Ausschnitt aus dem Strahlengang eines Peillichts;
- Fig. 3:: eine erfindungsgemäße Vorrichtung mit Darstellung des Strahlengangs des Peillichts;
- Fig. 4:: die Vorrichtung aus Fig. 3 mit Darstellung des Strahlengangs des Fixierlichts;
- Fig. 5:: eine alternative Ausführungsform einer erfindungsgemäßen Vorrichtung;
- Fig. 6:: eine erfindungsgemäße Vorrichtung bei der Verwendung;
- Fig. 7:: eine alternative Ausführungsform eines Peillichts;
- Fig. 8-10:: verschiedene Varianten von Bildern, die sich aus dem Peillicht und dem Fixierlicht auf der Retina ergeben können;
- Fig. 11:: eine alternative Ausführungsform des Strahlengangs für das erfindungsgemäße Fixierlicht.

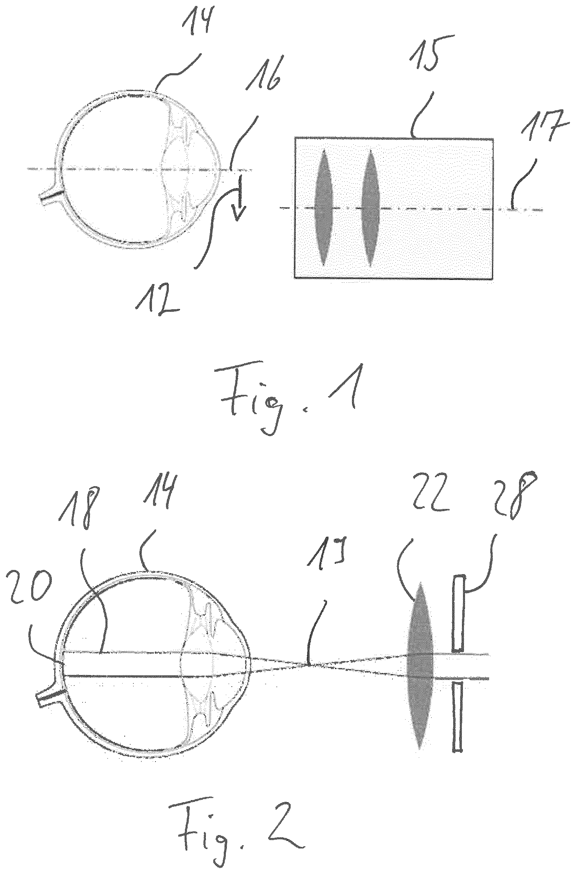

In Fig. 1 ist ein Auge 14 gezeigt, das vor einer erfindungsgemäßen Vorrichtung 15 angeordnet ist. Die Achse 16 des Auges 14 ist seitlich versetzt zu der Geräteachse 17, so dass das Auge 14 nicht richtig zu der Vorrichtung 15 ausgerichtet ist. Ziel des erfindungsgemäßen Verfahrens ist, dass Auge 14 in radialer Richtung 12 relativ zu der Vorrichtung 15 zu bewegen, so dass die Achse 16 des Auges 14 und die Geräteachse 17 zusammenfallen. Die Achse 16 des Auges 14 kann anfänglich eine von der Geräteachse 17 abweichende Richtung haben. Eine solche Abweichung wird automatisch ausgeglichen, sobald der Patient seinen Blick auf ein auf der Geräteachse 17 angeordnetes Fixierlicht richtet.

Gemäß Fig. 2 hat der Strahlengang eines Peillichts 18 einen Fokuspunkt 19, dessen Abstand zum Auge der Brennweite der optischen Elemente des Auges entspricht. Das Peillicht 18, dass in divergentem Zustand auf das Auge trifft, wird durch die optischen Elemente des Auges kollimiert und bildet auf der Retina 21 einen Fleck 20, der mit im Wesentlichen gleichmäßiger Helligkeit ausgeleuchtet ist.

Der Strahlengang des Peillichts 18 trifft in kollimiertem Zustand auf eine Linse 22, so dass der Strahlengang auf den Fokuspunkt 19 fokussiert wird. In dem kollimierten Abschnitt des Strahlengangs ist eine Blende 28 angeordnet, die den Strahlengang begrenzt, so dass sein Querschnitt beim Eintritt in das Auge kleiner ist als der Durchmesser der Iris, so dass der Strahlengang in seiner Gesamtheit durch die Pupille des Auges hindurchtritt. Die Blende 28 wirft einen Schatten auf die Retina 21 des Auges 14, so dass der Benutzer den Fleck 20 mit einer scharfen Umrandung 29 sieht.

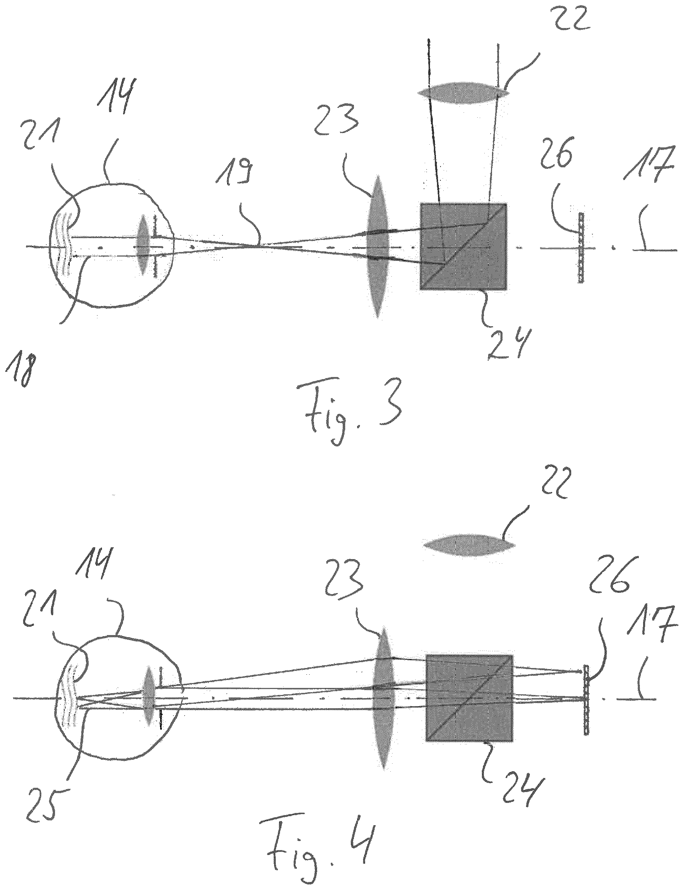

In einer erfindungsgemäßen Vorrichtung wird gemäß Fig. 3 ein kollimierter Lichtstrahl für das Peillicht 18 erzeugt, der mit einer ersten Linse 22 und einer zweiten Linse 23 auf den Fokuspunkt 19 fokussiert wird und dann in divergentem Zustand auf das Auge 14 trifft.

In einem Strahlteiler 24 wird der Strahlengang des Peillichts 18 koaxial mit dem Strahlengang eines Fixierlichts 25 überlagert. Der Strahlengang des Fixierlichts 25 geht aus von einem Display 26, auf dem ein Objekt 27 in Form eines Fadenkreuzes dargestellt ist. Das von dem Display 26 abgegebene Fixierlicht 25 tritt durch den Strahlteiler 24 und die zweite Linse 23 hindurch und wird in kollimiertem Zustand auf das Auge geleitet. Die zweite Linse 23 bildet also eine Abbildungslinse für den Strahlengang des Fixierlichts 25. Mit den auf unendlich adaptierten optischen Elementen des Auges 14 wird das Objekt 27 auf die Retina 21 des Auges 14 abgebildet. Der Benutzer sieht ein Bild des Fadenkreuzes.

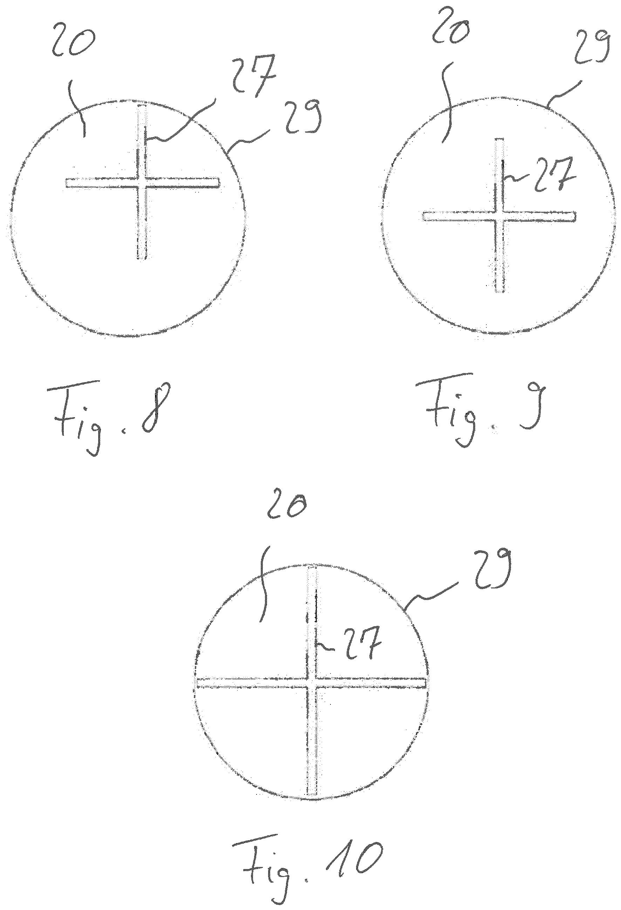

In Summe aus dem Strahlengang des Peillichts 18 in Fig. 3 und dem Strahlengang des Fixierlichts 25 in Fig. 4 sieht der Benutzer gemäß Fig. 8 einen hellen Fleck 20 mit einer scharfen Umrandung 29 sowie ein innerhalb der Umrandung 29 angeordnetes Bild des Objekts 27. Ist das Auge 14, wie in Fig. 1 gezeigt, nicht mit der Geräteachse 17 ausgerichtet, so sieht der Benutzer das Objekt 27 außermittig innerhalb der Umrandung 29, siehe Fig. 8. Wird das Auge 14 relativ zu der Vorrichtung 15 bewegt, so dass die Achse 16 des Auges 14 mit der Geräteachse 17 zusammenfällt, so sieht der Benutzer das Objekt 27 mittig innerhalb der Umrandung 29, siehe Fig. 9.

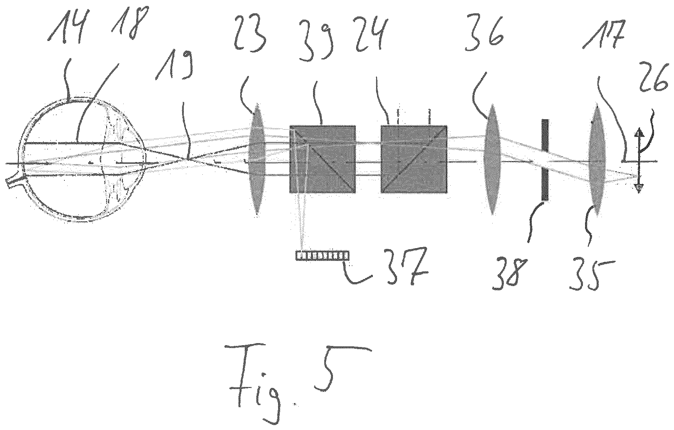

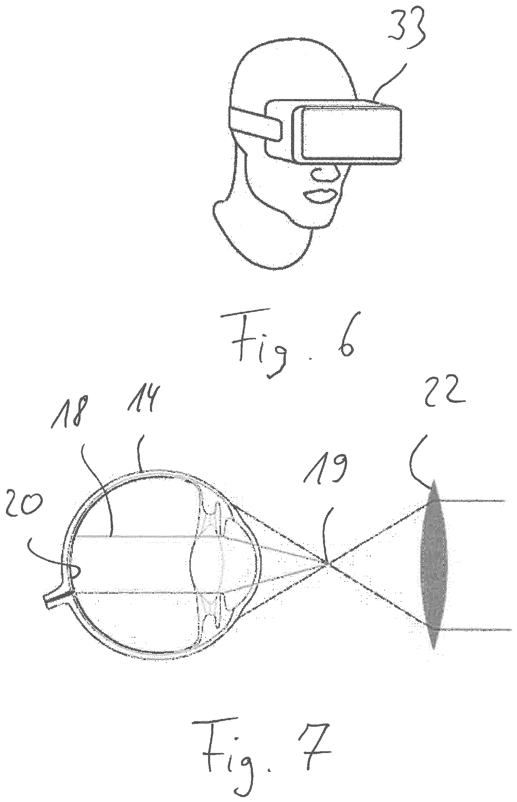

in Fig. 5, 6 ist eine erfindungsgemäße Vorrichtung in Form eines Full-Field-OCT-Geräts 33 dargestellt. Die Vorrichtung umfasst eine OCT-Lichtquelle, deren OCT-Licht aus der Austrittsfacette eines Diodenlasers austritt und auf das Auge 14 geleitet wird, sowie eine Auswerteeinheit, mit der vom Auge 14 zurückgestreutes OCT-Licht ausgewertet wird, um eine Bildinformation von der Retina zu gewinnen. Die Auswerteeinheit umfasst einen Bildsensor 37.In Fig. 6 ist das Full-Field-OCT-Gerät 33 bei der Verwendung gezeigt.

Bei der alternativen Ausführungsform gemäß Fig. 7 ist der Strahlengang des Peillichts 18 nicht mit einer Blende begrenzt. Der Strahlengang weitet sich ausgehend von dem Fokuspunkt 19 so weit auf, dass er die komplette Pupille des Auges 14 überdeckt und teilweise auf die Iris auftrifft. Die Iris begrenzt in diesem Fall den Strahlengang. Die Umrandung 29, die der Benutzer sieht, wird durch die Iris des Auges 14 erzeugt.

Bei einer weiteren Ausführungsform ist die Vorrichtung so eingerichtet, dass der Benutzer die Größe des auf dem Display 26 dargestellten Objekts 27 einstellen kann. Der Benutzer hat die Möglichkeit, die Größe des Objekts 27 so zu justieren, dass die peripheren Elemente des Objekts 27 in der Wahrnehmung des Benutzers mit der Umrandung 29 zusammenfallen, siehe Fig. 10. Dies ermöglicht eine präzise Ausrichtung des Auges 14 relativ zu der Geräteachse 17, weil der Benutzer nicht darauf angewiesen ist, die Mitte der Umrandung 29 zu schätzen.

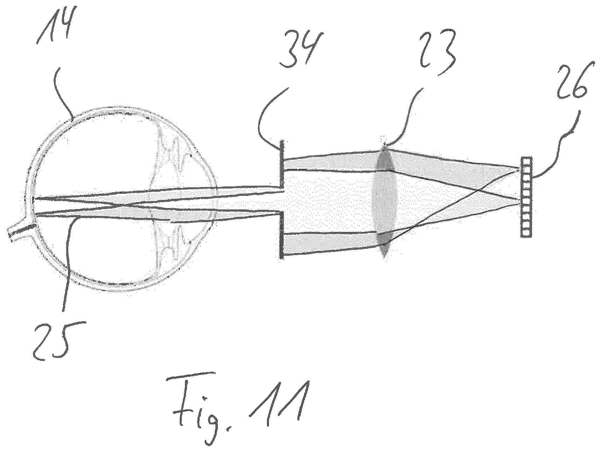

In Fig. 11 erstreckt sich der von dem Display 26 ausgehende Strahlengang des Fixierlichts 25 von dem Display 26 über die Kollimationslinse 23 zu dem Auge 14. Zwischen der Kollimationslinse 23 und dem Auge 14 ist eine Aperturblende 34 angeordnet.

Die Öffnung in der Aperturblende 34 hat einen Durchmesser von 0,5 mm, so dass die numerische Apertur des Strahlengangs, unter dem das Auge 14 das Objekt 27 auf dem Display 26 sieht, bei etwa NA = 0,015 liegt. Auf diese Weise wird das Objekt 27 auch für fehlsichtige Benutzer mit einer Fehlsichtigkeit zwischen +3 dpt. und -3 dpt. noch scharf dargestellt.

## Patentansprüche

1. Verfahren zum Ausrichten eines Auges (14) relativ zu einer Geräteachse (17) mit folgenden Schritten:
a. Erzeugen eines Fixierlichts (25), so dass das Fixierlicht (25) sich koaxial zu der Geräteachse (17) ausbreitet,;
b. Erzeugen eines Peillichts (18), so dass das Peillicht (18) sich koaxial zu der Geräteachse (17) ausbreitet und dass das Peillicht (18) eine von der Retina des Auges (14) beabstandete Fokuslage hat;
c. Bewegen des Auges (14) in radialer Richtung (12) relativ zu der Geräteachse (17), so dass mit dem Auge (14) eine Umrandung (29) des Peillichts (18) und das Fixierlicht (25) konzentrisch wahrgenommen werden.

2. Verfahren nach Anspruch 1, **dadurch gekennzeichnet, dass** das Fixierlicht (25) in Form eines ins Unendliche abgebildeten Objekts (27) auf das Auge (14) gerichtet wird.

3. Verfahren nach Anspruch 2, **dadurch gekennzeichnet, dass** das Objekt (26) des Fixierlichts (25) auf einem Display (26) dargestellt wird.

4. Verfahren nach Anspruch 2 oder 3, **dadurch gekennzeichnet, dass** das Objekt (26) mit einer numerischen Apertur nach unendlich abgebildet wird, die kleiner ist als 0,02.

5. Verfahren nach einem der Ansprüche 1 bis 4, **dadurch gekennzeichnet, dass** eine Aperturblende (34), die zwischen einer Abbildungsoptik (23) des Fixierlichts (25) und dem Auge (14) angeordnet ist, einen Durchmesser von weniger als 1 mm hat.

6. Verfahren nach einem der Ansprüche 1 bis 5, **dadurch gekennzeichnet, dass** das Peillicht (18) einen Fokuspunkt (19) aufweist, der in einem Abstand zwischen 10 mm und 25 mm zu der Vorderseite des Auges (14) angeordnet ist.

7. Verfahren nach einem der Ansprüche 1 bis 6, **dadurch gekennzeichnet, dass** der Strahlengang des Peillichts (18) so begrenzt ist, dass das Peillicht (18) die Pupille des Auges (14) nur zu einem Teil ausfüllt.

8. Verfahren nach einem der Ansprüche 1 bis 7, **dadurch gekennzeichnet, dass** der Durchmesser der Umrandung (29) des Peillichts (18) und/oder der Durchmesser des mit dem Fixierlicht (25) dargestellten Objekts (27) einstellbar ist.

9. Verfahren nach einem der Ansprüche 1 bis 8, **dadurch gekennzeichnet, dass** das Peillicht (18) eine Wellenlänge hat, die außerhalb des allgemein als sichtbar bezeichneten Wellenlängenbereichs liegt.

10. Verfahren nach einem der Ansprüche 1 bis 9, **dadurch gekennzeichnet, dass** das Auge (14) zu der Geräteachse (17) eines OCT-Geräts (33) ausgerichtet wird.

11. Vorrichtung zum Ausrichten eines Auges (14) relativ zu einer Geräteachse (17) mit einer ersten Lichtquelle (30) zum Erzeugen eines Peillichts (18), wobei der Strahlengang des Peillichts (18) sich von der ersten Lichtquelle (30) zu dem Auge (14) erstreckt, so dass das Peillicht (19) eine von der Retina (21) beabstandete Fokuslage hat, und mit einer zweiten Lichtquelle (26) zum Erzeugen eines Fixierlichts (25), so dass das Fixierlicht (25) aus dem Unendlichen abgebildet auf das Auge (14) trifft.
